# EUROPEAN PATENT APPLICATION

(11) **EP 4 356 952 A1**
(43) Date of publication of application: **24.04.2024**
(21) Application number: 22202550.4
(22) Date of filing: 19.10.2022
(51) Int. Cl.: A61N 1/04, A61N 1/36, A61N 1/372

(54) **SYSTEM FOR PRODUCING SOMATOTOPIC SENSATIONS USING TRANSCUTANEOUS ELECTRICAL NERVE STIMULATION (TENS)**

(71) Applicant: ETH Zürich, 8092 Zürich (CH)
(72) Inventor: RASPOPOVIC, Stanisa, 8006 Switzerland (CH); VALLE, Giacomo, 3011 BERN (CH); CHEE, Lauren, 8057 ZÜRICH (CH); CIMOLATO, Andrea, 8049 ZÜRICH (CH)
(74) Representative: ZBM Patents - Zea, Barlocci & Markvardsen

(57) **Abstract**

System for producing somatotopic sensations using Transcutaneous Electrical Nerve Stimulation (TENS) in the lower limbs is provided, the system comprising a wearable device to be attached to a lower limb of an individual, the wearable device comprising at least two units, each comprising one or more arrays of TENS electrodes to be associated with at least one nerve of the lower limb, and wherein the system is configured to select electrodes of at least one array of electrodes, and stimulation signal parameters to be applied thereto, and control the stimulation of at least one nerve associated with the array of electrodes comprising the selected electrodes.

## Description

### BACKGROUND

Individuals with peripheral sensory loss due to peripheral neuropathy or amputations are affected by reduced mobility, impaired locomotion, and pain. Transcutaneous electrical nerve stimulation (TENS) has been shown to successfully stimulate nerves upstream of where sensations are perceived, through external skin electrodes. Furthermore, this technology has sparsely been used to artificially restore tactile sensations from the location where the sensation was lost (i.e., somatotopic sensations corresponding to, for example, limbs affected by a neuropathy, or amputated limbs), such as in cases of transradial or transtibial amputees.

However, these examples required the constant supervision of an expert for electrode placement and calibration of stimuli applied through the electrodes. More specifically, variations of nerve trajectory, body size or tissue composition differing between individuals make accurate electrode placement difficult. Even with the intervention of highly specialized medical personnel, with deep knowledge of neuroanatomy, some somatotopic sensations feel unnatural to the individual and are difficult to repeat or reproduce between different sessions. The unnaturalness is due to non-biomimetic stimulation, which is a result of imprecise electrode placement and simplistic or inaccurate stimulation patterns. Furthermore, body movement, which may cause electrodes to change position relative to subcutaneous structures like nerves, and perspiration, can lead to changes in electrode conductivity. This results in different activations of the nerve over time, in response to the same calibrated nerve stimulation.

Therefore, not only the initial calibration process is time consuming, requiring expert decision making before every session, but also the stimulus delivery needs to be adapted over time (by recalibrating the stimulation) and even, for example, during movement. Additionally, the pathology progress or the benefits of a treatment strongly influence the characteristics of the stimulation required by the user in the long term.

Therefore, there is a need for a device, especially to be employed for the lower limbs of an individual, which pose more problems than upper limbs due to the position of the nerves, that is able to solve the above-mentioned problems, enabling a user-specific set up, accurate positioning of the electrodes with respect to the nerves, and a calibration which can be performed by the user, without the presence of an expert.

### SUMMARY

In a first aspect, a system for producing somatotopic sensations using Transcutaneous Electrical Nerve Stimulation (TENS) in the lower limbs is provided, the system comprising:
- a wearable device to be attached to a lower limb of an individual, the wearable device comprising:
   - at least two units, each unit comprising an array of TENS electrodes to be associated with at least one nerve of the lower limb; and
   - adjustable fasteners to attach the units together and to the lower limb;
- a controller in communication with a stimulation module which is connected with the electrodes for generating electrical signals to be applied to the electrodes, the controller being configured to:
   - select at least one pair of electrodes of at least one array of electrodes, and select stimulation signal parameters to be applied to the selected at least one pair of electrodes; and
   - control the stimulation module to generate and send, to the selected at least one pair of electrodes, an electrical signal based on the selected stimulation signal parameters, to stimulate at least one nerve associated with the array of electrodes comprising the selected at least one pair of electrodes.

The stimulation module may be part of the system, or it may be an external device, e.g. a conventional stimulator used in a hospital or other facility, that is connected to the system according to the invention, i.e. connected to the controller and to the electrodes.

In embodiments of the present disclosure, some elements of the stimulation module may be external, and may be provided or sold separately from the system, and other elements of the stimulation module may be an integral part of the system. For example, a stimulator for generating electrical signals may be separate device, connected to the system but that is not part of the system, while suitable electronic circuits for transmitting the electrical signals generated by the stimulator to selected electrodes (as described later on in the present disclosure) may be part of the system.

Each pair of electrodes selected to receive electrical signals, and therefore apply stimulation to the associated nerve or nerves, may comprise a cathode and an anode.

Each of the cathode and anode may have any suitable geometry, and the cathode and anode may be arranged one relative to the other as convenient in each particular case or embodiment, e.g. concentrical to each other, side by side, or in other configurations. A "pair of electrodes" may also be formed herein by an active electrode and a ground electrode. The ground electrode may be common to several active electrodes, and therefore to several pairs of electrodes. In some examples, one electrode of an array of electrodes may be selected as cathode, and one or more electrodes of the array of electrodes may be selected as anodes.

The wearable device may be in the form of, for example, a composite brace, i.e., a brace formed of several parts, that can be worn around one of the lower limb joints. More precisely, such wearable device, such as a knee or ankle brace (for example, a "sock"), may be anatomically personalized to be wrapped around a limb such as, for example, a knee above a stump, or an ankle of an individual suffering from neuropathy (i.e., the individual has a partial or total loss of sensations from the foot, ankle, etc,), such that it adopts an accurate position with respect to a joint of the individual. The at least two units of the wearable device may be independently placeable units, intended to be placed around a lower limb and attached to each other with the adjustable fasteners.

The wearable device may be configured to cover, when placed on the skin of an individual at a lower limb of the individual, one or more nerves or nerve branches that are under the skin of the individual in the area of contact with the wearable device. The at least two units of the wearable device allow accurately covering such nerves or nerve branches in a lower limb of a specific individual. Each unit comprises an array of TENS electrodes, e.g. an array of pairs of electrodes, each pair comprising an anode and a cathode, which is to be associated with at least one of the nerves of the lower limb, and adjustable fasteners to attach the units together and to the lower limb in a specific position. This configuration is further detailed below.

The array of TENS electrodes comprises a plurality of electrodes that may have different sizes. Furthermore, the number and disposition of the electrodes may vary from one array to another. Having various sizes, amount, and dispositions implies that the electrodes may be disposed in an array in different directions with respect to the electrodes in another array or unit of the wearable. Electrodes of different sizes disposed in array around at least one nerve, in combination with the adjustable fasteners, allow reaching and maintaining a precise positioning of the unit suitable to stimulate the nerves of the lower limbs.

The electrodes may be for example dry gold-plated electrodes, silicone conductive rubber, foam-based electrodes, wet Ag/AgAI electrodes, or other. For the electrical current flow, a pair of electrodes may comprise an anode electrode and a cathode electrode arranged in any layout, e.g. side by side, or with an active electrode and a corresponding ground electrode arranged concentrically. The array of electrodes may comprise a common cathode or ground electrode for the electrodes of an array, or each electrode may have a corresponding cathode or ground electrode.

In the present disclosure the expression "array of electrodes" means a group of electrodes placed, according to a regular or irregular arrangement, in an area of the wearable unit.

The controller may select a set of stimulation signal parameters, such as the frequency, pulse width and amplitude of the signal to be generated by the stimulation module and applied to the selected electrodes, which may be in communication with the controller both directly or indirectly, for example through a switch and transmission lines, to stimulate the nerve or nerves associated with the selected electrodes, in order to produce a specific somatotopic sensation in the user.

In this way, a specific nerve or group of nerves, i.e., one or more nerve branches, can be targeted, and a signal can be applied therein, eliciting a desired somatotopic sensation in the individual, who recognizes such sensation from an affected limb or even from a missing limb. The system further allows to apply the best combination of stimulation parameters, such as activation/deactivation of the electrodes in the arrays, frequency, pulse-width and amplitude of the stimulations, that produce artificial sensory feedback close to a natural one or close to intended sensory feedback.

Systems according to the present disclosure also have the ability to modulate in real time the stimulation to the nerves, e.g. by receiving inputs from external sensors, as will be disclosed later on.

Furthermore, the anatomically personalized wearable device may be easy to wear, comfortable, washable, and reusable.

The configuration of the wearable device with two units, and adjustable fasteners, guarantees that the electrode arrays are placed directly over the anatomical location where specific, and in some cases the most important, nerves or nerve branches are nearer the surface of the skin, even in case of e.g. body movements which could otherwise cause electrodes to change position relative to subcutaneous structures like nerves. The fasteners also assist in the correct placement of the wearable device even when the apparatus is used by a naive user, thus removing the need of an expert for supervision of the correct maintenance of electrode placement.

Finally, the presence of electrode arrays, wherein an array comprises one or more pairs of electrodes, allows selected electrodes to be activated, and also multipolar (i.e., simultaneously in multiple electrodes) and time-variant activation of the electrodes, and therefore also guarantees the possibility of personalizing the stimulation to account for different anatomies and nerves trajectories underneath the patch of skin.

### BRIEF DESCRIPTION OF THE DRAWINGS

Non-limiting examples of the present disclosure will be described in the following, with reference to the appended drawings, in which:
Figures 1 and 2 show two different views of a first example of the system according to the present disclosure, adapted for a foot of an individual.
Figures 3 and 4 show two different views of a second example of the system according to the present disclosure, adapted for a knee of an individual.
Figures 5A, 5B and 5C show three different perspectives of electrode arrays of the system corresponding to the first example, and the nerves passing through the foot of the individual.
Figure 6 depicts a diagram block of the example of the system of figure 1 and 2.
Figure 7 depicts a flow chart of a calibration process of an example of the system according to the present disclosure.
Figures 8, 9, 10 and 11 depict diagrams of stimulus generation of an example of a system according to the present disclosure, while being in operation.
Figure 12 depicts a flow chart of a stimuli process of an example of the system according to the present disclosure.

### DETAILED DESCRIPTION OF EXAMPLES

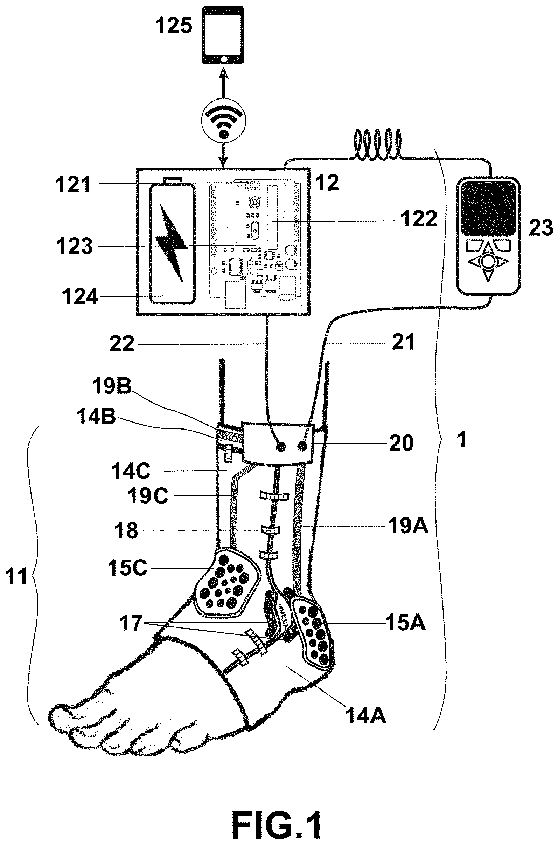

Figure 1 shows an example of a system according to the present disclosure, wherein the system 1 has two components: the leg brace 11 and a controller or control box 12. The system may also comprise a stimulator 23 for generating electrical signals, although the stimulator 23 may also be external to the system and connected to it when necessary.

The stimulator 23 is in communication with the control box 12 and connected with a switch 20. The stimulator 23 and the switch 20 may form, or belong to, a stimulation module, such a stimulation module operating under the control of the control box 12 to generate electrical signals and apply the generated electrical signals to selected electrodes, or pairs of electrodes, that are connected to the switch, as will be disclosed in more detail below. In some embodiments, the switch 20 of the stimulation module belongs to the system, and is e.g. integrated or attached to the leg brace 11 or other wearable device according to the present disclosure, while the stimulator 23 is an external simulator, and does not belong to the system.

In other words, even though a stimulator such as 23 and a switch such as 20 are both parts of a functional unit for providing electrical signals to the electrodes to perform the nerve stimulation, which is herein referred to as "stimulation module", part of this functional unit, in particular the stimulator, may be provided separately and attached to the system when needed, or may be integrated in the system.

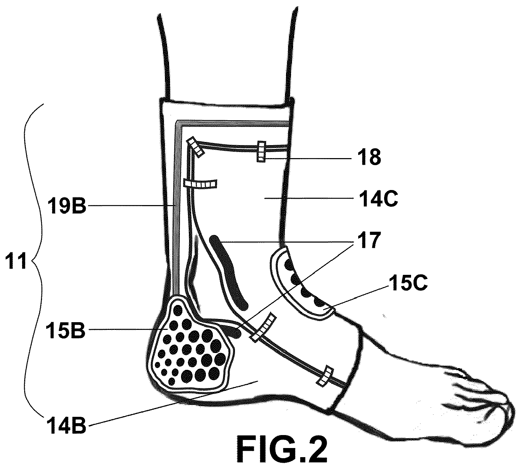

The leg brace 11 comprises three units shown in figure 1: unit 14A, unit 14B, and unit 14C. Each unit 14A, 14B, and 14C is built with elastic fabric and incorporates at least one array 15A and 15C of biocompatible electrodes for TENS shown in fig.1 and at least a third array 15B not shown in figure 1, but shown in figure 2. The array 15C comprises biocompatible electrodes of different sizes, for which the distance between the electrodes thereof varies. Additionally, the number of electrodes of the biocompatible array 15A and 15C may be different. The units allow providing a composite anatomically personalized leg brace. Each electrode array is flexible, washable, and directly integrated into the elastic fabric of the unit. In the case of figure 1, the electrode arrays may be sewed on the fabric of each corresponding unit. As can be seen in figure 1, the units 14A and 14C further include joint-molded anchoring pads 17 to ensure correct positioning of the brace around the joint, in the case of figures 1-2 being the ankle joint, in a specific and repeatable way, so the electrode arrays are associated with specific nerves. "To ensure correct positioning" may be understood, in the present disclosure, by "to guide a user for positioning the wearable device or brace in a correct position on a limb, for example on a knee, or on an ankle". As can be seen in figure 2, also the units 14B and 14C include joint-molded anchoring pads 17. Joint-molded anchoring pads may comprise pads, thicker and slightly more rigid than the rest of the fabric of the wearable device, that are shaped to surround part of a joint. Each unit of the wearable may comprise anchoring pads, each anchoring pads shaped to surround part of a joint, so in the units may be positioned more spaced apart from each on users with larger joints than in users with small joints, making the brace customizable. The anchoring pads and the design of each unit allow, therefore, that the electrode arrays are positioned in an area of the limb in correspondence with the targeted nerves The position for applying stimuli is then fine-tuned to each user by selecting, during a calibration or training routine, as explained further below, electrodes of the array that are "closer" to each specific nerve.

The anchoring pads may adopt the form of part of a contour of a joint or bone. Each unit 14A, 14B, and 14C can be adjusted to the user anthropometry using adjustable fasteners 18 connecting them. The fasteners may be Velcro straps, or similar fasteners, and may comprise self-sizing adjustable fasteners. In this example, the specific characteristics of each unit 14A, 14B, and 14C (making the wearable device self-adjusting and/or elastic) and the presence of anchoring pads guarantee that the electrode arrays 15A, 15B (15B not shown in figure 1 but shown in figure 2), and 15C are placed in specific anatomical locations over where major peripheral nerves branches (wherein a sensation is lost) are near the surface of the skin. The anchoring pads facilitate the naive user to identify the correct positioning of the device. The electrode arrays 15A, 15B, and 15C are connected to a switch 20 through conductive lines 19A,19B, and 19C, which may be attached to or embedded in the fabric of the corresponding unit, in such a way to adapt to the movements of the fabric. In some implementations the conductive lines may be implemented as flexible conductive lines of sufficient length that allows for movement of the joint without tensioning the lines. The movement of the lower limb is therefore facilitated and the need of external conductive lines is avoided, reducing encumbrance. Other kinds of conductive lines may be used by other examples. The switch may be part of the stimulation module and may be connected to the controller, and a plurality of transmission lines, e.g. conductive lines, may individually connect each electrode to the switch, wherein the switch is configured to send to at least one pair of electrodes, according to a selection received from the controller, an electrical signal received from the stimulator 23. The switch may comprise or may be a channel selecting Printed Circuit Board (PCB). The switch or channel selecting PCB may be on the wearable device, so conductors may be present between the controller/stimulator and the wearable device. The switch may also be physically integrated in the control box, but preferably the switch may be attached to one of the units of the wearable device, or to several units in a region where the different units are in contact (see e.g. figure 1), for example removably attached. Each unit of the wearable device may comprise transmission lines connected to the switch and to each electrode of the arrays of electrodes of the unit, with suitable connectors if needed.

With reference to figure 1, the switch 20 allows transmitting signals from connection lines 21 and 22 towards the electrodes in the electrode arrays through the conductive lines 19A, 19B, and 19C, or conductive bus lines. In some examples the transmission lines may be flexible and/or elastic conductive lines. In some examples, an external stimulator 23 transmits a stimulation signal to the switch and the control box 12 transmits a selection signal comprising data related to a selected at least one pair of electrodes to the switch 20. The switch may then activate only the selected at least one pair of electrodes and send the stimulation signal only to the selected electrodes. The switch 20 may be attached to one of the units of the wearable device, and each unit of the wearable device may comprise transmission lines connected to the electrodes of the arrays of electrodes of the unit. In the present disclosure, a stimulator may be understood as a device capable of generating electrical signals to be applied to the electrodes, e.g. through a switch, as explained. The system may be used at home and/or during normal life by a user, and the stimulation module comprising the stimulator and the switch may then be integrated in the system. The system may also be used for therapeutical purposes or intended to be used in a hospital, connected to an external stimulator.

The control box 12 comprises a communication unit 121, a controller 122, a storage unit 123, and a battery supply 124. The controller 122 within the control box 12 communicates through the communication unit 121 with an external generic User Interface (Ul) 125 (in this case, an app within a tablet or smartphone).

Figure 2 shows a medial plane perspective of the leg brace 11 of the example of figure 1, wherein the units 14B and 14C are shown placed on an opposed side of the ankle, the unit 14B comprising an electrode array 15B. As seen in figure 1, each electrode of each electrode array is connected to the switch 20 through conductive lines. Figure 2 shows the electrode array 15B connected to a conductive line 19B. The conductive line 19B comprises multiple conductors, e.g. one for each electrode of the array, and is to connect to the switch 20 (not shown in figure 2 but shown in figure 1). Figure 2 further depicts joint-molded anchoring pads 17 to ensure correct positioning of the brace 11 around the joint. Also, further self-sizing adjustable fasteners 18 are shown connecting units 14B and 14C.

In the example shown in figures 1 and 2, the stimulator 23 and the switch 20 are connected to the controller 122, in such a way that the controller 122 is configured to control the stimulator module, i.e. the stimulator 23 and switch 20. In the present disclosure, "to control the stimulation module" may comprise sending commands and/or information comprising selected stimulation signal parameters to the stimulation module so that the stimulation module generates and sends a specific electrical signal based on the selected stimulation signal parameters to the electrodes. The system is, in this way, configured to stimulate at least one nerve associated with the array of electrodes comprising the selected at least one pair of electrodes. In figures 1 and 2, the controller 122 controls the stimulator 23 by sending to the stimulator 23 a set of stimulation signal parameters. In response to receiving such stimulation signal parameters, the stimulator 23 outputs an electrical signal through connection line 21, which is sent to the switch 20. In parallel, the controller 122 is further connected to the switch 20, via connection line 22, the controller being configured to send data which allows selecting at least one pair of electrodes of at least one array of electrodes to the switch 20. By receiving such data, switch 20 can redirect the received electrical signal from stimulator 23, to one or more specific electrode or pair of electrodes, using the corresponding bus lines 19A, 19B or 19C.

As seen, the examples of figures 1 and 2 relate to a system wherein the wearable device is intended to be worn around an ankle of a user and comprises three units. Each of the units comprises one array of electrodes, wherein the array of electrodes of a first unit is to be associated with the Peroneal Nerve and may comprise between 2 and 9 electrodes. The diameter of each electrode may be comprised between 1 and 4 cm. The array of electrodes of a second unit is to be associated with the Tibial Medial Nerve and may comprise between 2 and 17 electrodes, the diameter of each electrode being between 1 and 4 cm. The array of electrodes of a third unit is to be associated with the Calcaneal Nerve and comprises between 2 and 9 electrodes, the diameter of each electrode being between 1 and 3 cm. In some cases the stimulation of the electrodes for a wearable device for the ankle may be performed with electrical signals having values in the following ranges: range of injected charge for the Peroneal Nerve 1 to 15 µC, for the Tibial Medial Nerve 0.8 to 20 µC, for the Calcaneal Nerve 0.8 to 15 µC; range of stimulation frequency for the Peroneal Nerve 40 to 150 Hz, for the Tibial Medial Nerve 40 to 150 Hz, for the Calcaneal Nerve 40 to 150 Hz.

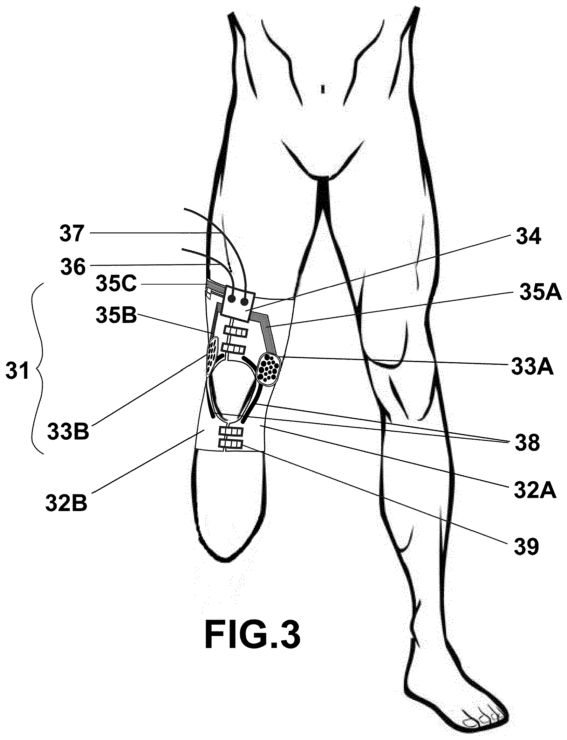

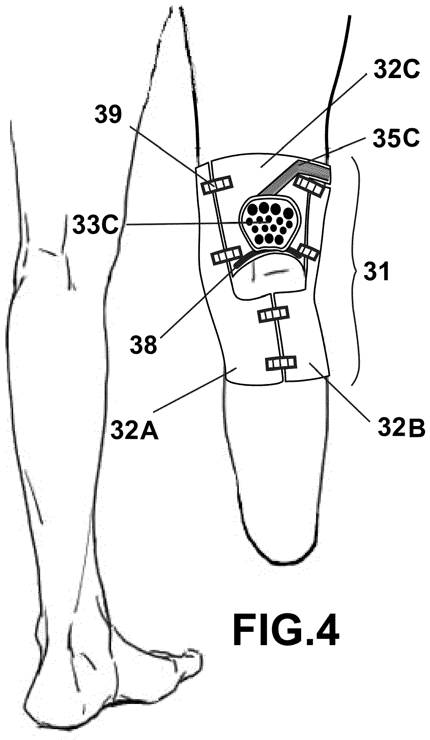

Figures 3 and 4 show a different example of a system according to the present disclosure, wherein a wearable in the form of a knee brace is depicted, being worn by a patient with an amputated leg. More precisely, figure 3 shows a frontal plane view of a leg brace 31 (in this example, a specific knee brace 31) comprising three units 32A 32B and 32C. Each unit is also built with elastic fabric and incorporates at least one array 33A, 33B and 33C of biocompatible electrodes for TENS. Each electrode of each electrode array 33A, 33B and 33C is connected to a switch or channel selecting Printed Circuit Board (PCB) 34 through conductive lines 35A, 35B, and 35C respectively. The switch or channel selecting PCB 34 allows direct connection lines 36 and 37 to both an external or integrated stimulator and the control box. The stimulator and control box (not shown for this example) are like stimulator 23 and control box 12 of Figures 1-2. The units integrate joint-molded anchoring pads 38 also referred to as joint anchors 38, to ensure correct positioning of the brace 31 around the knee joint. The joint anchors are shaped to position the unit with respect to a joint of the lower limb in a specific and repeatable way, in the case of figure 3 being a knee joint, associated with specific nerves. Each unit can be adjusted to the user anthropometry using adjustable fasteners 39 connecting them. The fasteners may be self-sizing adjustable fasteners.

In figure 3 the joint anchor comprises a pad arranged along a contour portion of the unit and has a shape matching at least part of the knee joint. As it may become apparent, each unit of the wearable device may comprise one or more joint anchors, wherein joint anchors of different units may be configured to at least partially surround between them a joint of the lower limb, when the wearable device is attached to the lower limb.

Figure 4 shows a rear view of the leg brace 31 shown in the example of figure 3 and the three units 32A, 32B and 32C can be seen. In this view, the electrode array 33C can also be seen in unit 32C, as well as the conductive line 35C. The conductive line 35C is to connect the array 33C with the switch or channel selecting Printed Circuit Board (PCB) 34 (not visible in figure 4). Furthermore, unit 32C also comprises the joint-molded anchoring pad 38 to ensure correct positioning of the brace around the knee joint, and the adjustable fasteners 39 connecting unit 32C to units 32A and 32B.

As seen, the system of figures 3 and 4 comprise a wearable device intended to be worn around the knee of a user. The wearable of the knee comprises three units, each comprising one array of electrodes. The array of electrodes of a first unit is to be associated with the Frontal Tibial Nerve and comprises between 2 and 16 electrodes, the diameter of each electrode being between 1 and 3 cm. The array of electrodes of a second unit is to be associated with the Common Peroneal Nerve and comprises between 2 and 16 electrodes, the diameter of each electrode being between 1 and 3 cm. The array of electrodes of a third unit is to be associated with the Posterior Tibial Nerve and comprises between 2 and 16 electrodes, the diameter of each electrode being between 1 and 2 cm. In some cases the stimulation of the electrodes for a wearable device for the knee may be performed with electrical signals having values in the following ranges: range of injected charge for the Frontal Tibial Nerve 0.5 to 5 µC, for the Common Peroneal Nerve 0.2 to 11 µC, for the Posterior Tibial Nerve 0.8 to 9 µC; range of stimulation frequency for Frontal Tibial Nerve 40 to 150 Hz, for the Common Peroneal Nerve 40 to 150 Hz, for the Posterior Tibial Nerve 40 to 150 Hz.

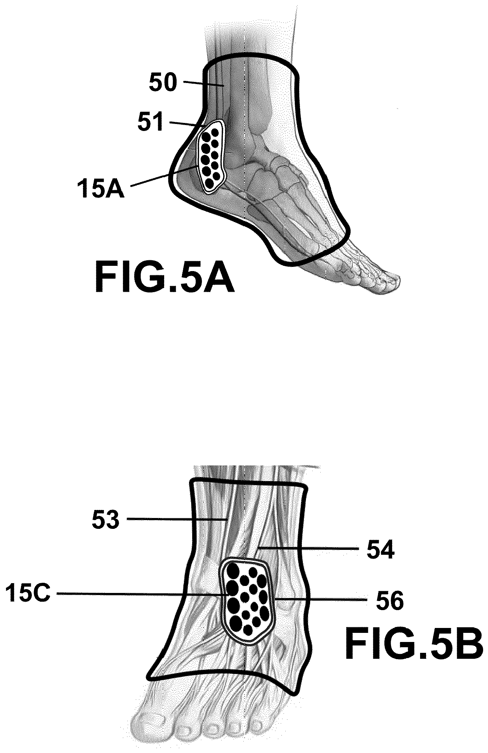

Figure 5A shows a view of the wearable device of the example depicted in figures 1 and 2, i.e., a wearable sock, wherein the specific characteristics of each unit guarantee that the electrode array 15A is placed in such a way that it is associated with the Peroneal Nerve 50 and the saphenous vein 51 of the lower limb of the user. Therefore, ensuring placement of array 15A in such specific anatomical location over where the Peroneal Nerve branch is near the surface of the skin allows a more precise stimulation thereof.

Figure 5B shows a different view of the same wearable sock of the example depicted in figures 1 and 2, wherein electrode array 15C is located over the Deep Fibular Peroneal Nerve 53, Medial Dorsal Cutaneous Nerve 54, and Intermediate Dorsal Cutaneous Nerve 56, which are all branches of the Peroneal Nerve. Again, the same specific characteristics of the sock guarantee that the electrode array 15C is placed over where said nerve branches are near the surface of the skin.

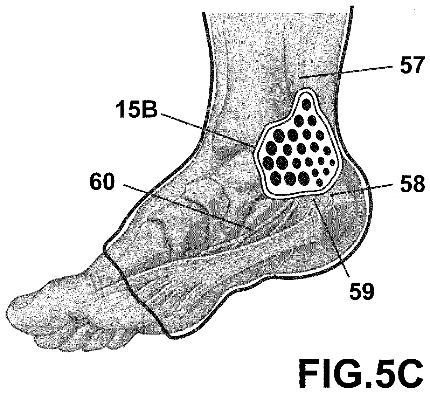

Figure 5C shows a further different view of the same wearable sock of the example depicted in figures 1 and 2, wherein electrode array 15B is located over the calcaneal branches of the Tibial Nerve 57, namely Posterior Tibial Nerve 57, Calcaneal Nerve branch 58, Lateral Plantar Nerve 59, and Medial Plantar Nerve 60. Again, the same specific characteristics of the sock guarantee that the electrode array 15B is placed over where said nerve branches are near the surface of the skin.

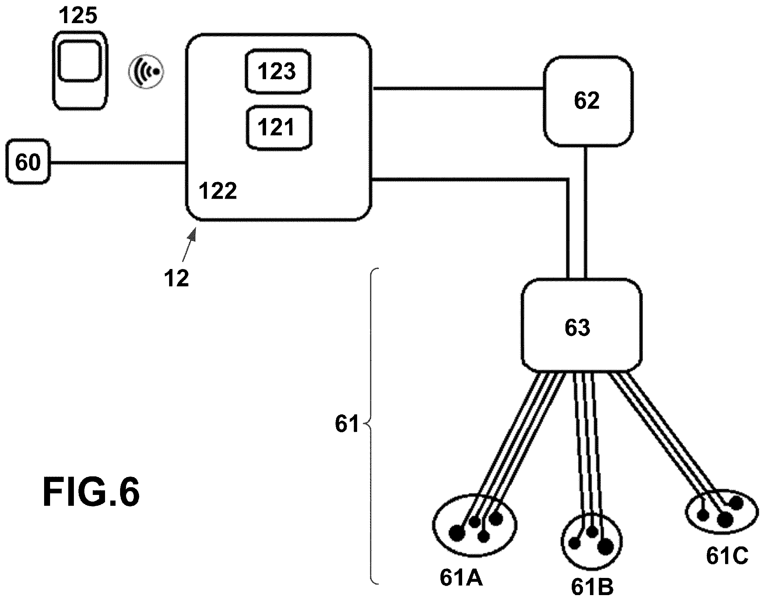

According to some examples, the system may comprise a user interface in communication with the controller, and a storage device may be connected to the controller. In particular, figure 6 depicts a diagram block of the example of the system of figures 1 and 2, wherein the control box 12 comprises a controller 122, a communication unit 121, and a storage unit 123. This figure depicts the layout of an example of the system, and the flow of information in and out of the control box. The controller 122 communicates through the communication unit 121 with a User Interface (Ul) 125 (in this case, an app within a tablet or smartphone). Through the UI 125, the controller 122 may guide the users through a machine learning-driven calibration of the stimulation of certain nerves, and, in response, collect their feedback. The machine learning-driven calibration is performed by using the controller 122. In addition, the communication unit 121 can collect sensor information from sensors 60, which may be used by the controller 122 to control the generation of the stimuli. More precisely, the controller 122 may send a control signal to the stimulator 62, which may generate the electrical signals and send them to a switch 63. Furthermore, the controller 122 sends a control signal to the switch 63 which, in response, may switch the electrical signal received from the stimulator 62, sending it to a certain group of electrodes of a specific electrode array 61A, 61B or 61C.

The storage unit 123 is responsible for collecting data produced by the controller 122 (the results of the calibration, in the form of an intended user sensation and its corresponding stimulation signal parameters and group of selected electrodes).

Furthermore, in use, after the calibration of the system is performed, the controller 122 may also send data in order to control the performance of the switch or channel selecting PCB 63 (for example, a list of active and non-active electrodes), determining which electrode in the arrays should be active during the stimulation, while the controller 122 may send a control signal to the external stimulator 62, to generate the electrical signal to perform the nerve stimulation.

According to a specific example, the controller may be configured to calibrate the system for one user, for producing at least one intended user sensation, by performing, for a specific intended user sensation, a first calibration cycle, the first calibration cycle comprising:
- selecting at least one pair of electrodes of at least one array of electrodes;
- causing a nerve stimulation by applying an electrical signal to the selected at least one pair of electrodes to stimulate at least one nerve, the electrical signal being based on predetermined stimulation signal parameters;
- receiving from the User Interface feedback data relating to a sensation experienced by the individual in response to the nerve stimulation; and
- based on the received feedback data, storing in the storage device:
   - the intended user sensation;
   - data related to the selected at least one pair of electrodes; and
   - the predetermined stimulation signal parameters.

The system may be calibrated for a desired intended user sensation. The calibration may be performed for a specific user and the calibration may be made by a non-expert user. The intended user sensation may be related to a movement of a limb of the user, either from an existing limb which is affected by a neuropathy, or from a non-existent limb, in the case of amputees. For example, a user with a neuropathy-affected foot may want to calibrate the system to produce somatotopic sensations according to a specific movement of the foot, for example, the ankle of the foot being flexed forwards, and the tip of foot touching the floor, wherein the toes should be touching the floor.

Therefore, an intended user sensation may be chosen and, for example, inputted through a user interface, such as a specific device with an interface, or a smartphone with an app in communication with the controller of the system, in the form of a tag corresponding to such movement. In this way, the system may obtain the intended user sensation, and the system may select a specific combination of electrodes, from one or more of the arrays of the wearable, and stimulation signal parameters to apply an electrical signal thereto.

The electrical signal may produce a nerve stimulation to a nerve or nerve branch or branches corresponding to the electrodes position on the limb, and, in response, the user may input, through the User Interface, feedback data relating to a sensation he/she is experiencing. In the case where inputted data describes a sensation similar to the intended user sensation, such data may be stored within the storing device, associated to the pair of electrodes used to apply the electrical signal and the parameters related to the signal. The data inputted by the user according to the sensation may be within a range of similitude, or within a range of tolerance, to the intended user sensation: different options may be used by the system such as a scale of similitude with a sensation, by presenting a question to the user, for example: "how close is your sensation to feeling your ankle flexing upwards in a scale from 1 to 10?".

A somatotopic sensation (ankle flexion; ankle extension; heel touch; etc.) may be evoked by stimulating one or multiple selected pairs of electrodes of at least one array. In this case, for one specific user and one specific sensation, the same signal may be applied to all the selected pairs of electrodes of the array, or the signal parameters may be different for each pair of electrodes.

The system may implement an artificial intelligence, Al, -driven calibration that selects electrode configurations and defines electrical stimulations parameters (e.g., amplitude, frequency, and pulse-width) to evoke a natural somatotopic sensation.

Advantageously the system is able to self-calibrate based on user feedback data. The self-calibration may be advantageous for everyday use so that similar or same sensations may be easily re-elicited day after day. Long-term changes in nerve conductivity or morphology of a user may affect the characteristics of neuromodulation. The system of the present disclosure may allow avoiding investing extra time and labor for recalibration. In these cases, the system comprising a composite anatomically personalized limb interface with embedded TENS electrodes arrays provides an Al-driven or machine learning-driven calibration function for multipolar and biomimetic stimulations that guarantees the naturalness of the somatotopic evoked sensations. In the present disclosure multipolar stimulation may refer to simultaneously stimulating multiple electrodes of one array, to adapt the stimuli to the precise position of the nerves of a user.

In some embodiments, the goal of the calibration is to achieve a perceivable, somatotopic, and comfortable sensation in each of the areas innervated by the target nerves. Indeed, it is first required that the electrically evoked sensation is clearly perceived (above perceptual threshold) and that an intensity modulation is identified. Second, it is important to evoke a somatotopic sensation (sensation perceived directly on the are innervated by the target nerve) that is inherently intuitive, allowing for immediate and effortless understanding of the feedback for each of the nerves. The area of somatotopic elicited sensation has to be as large as possible without overlaps with the other evoked sensations from the other stimulation points. Third, the sensation has to be comfortable, as natural as possible, and not painful.

The first calibration cycle may comprise an initialization step which may comprise input of biological data (e.g., age, height, weight). Sub-sequent calibration cycles may be implemented, as explained further below.

According to a particular example, the controller may be further configured to perform subsequent calibration cycles, wherein each subsequent calibration cycle comprises:
- causing a nerve stimulation by applying an electrical signal of selected stimulation signal parameters to at least one selected pair of electrodes of the array of electrodes, wherein the at least one selected pair of electrodes and/or the stimulation signal parameters are different from those of a previous cycle;
- receiving from the User Interface feedback data relating to a sensation experienced by the individual in response to the nerve stimulation; and
- based on the received feedback data, storing in the storage device:
   - the intended user sensation;
   - data related to the at least one selected pair of electrodes; and
   - the stimulation signal parameters.

Each implementation of subsequent calibration cycles or training routine may generate a different electrode combination in the arrays and different modulation strategies. Stimulation may then be applied to the user and feedback about the perceived sensation is collected through the Ul. This information may then be transmitted to an Al-calibration algorithm. Calibration cycles or subsequent calibration cycles may be implemented until restored sensory feedback results are satisfactory for a user or other loop-breaking conditions are met. Calibration cycles or subsequent calibration cycles may be implemented to restore multiple somatotopic sensations. The stimulation-specific combination of electrodes in the array and the modulation parameters are finally saved in the memory unit. At the end of the calibration the user may be in possession of a system able to subcutaneously stimulate specific areas for the restoration of somatotopic sensation on an ankle and /or foot.

During calibration, for each somatotopic sensation, there may be a number of different combinations of electrodes in the array, and different signal parameters, that the system may generate and apply during a calibration cycle to the user. The system may also collect the user feedback. The initial combination of electrodes and signal parameters may be pre-programmed, and may depend, as said, on a user's biological data. The calibration cycle then may generate subsequent combinations of electrodes and signal parameters driven by the user feedback and may be based on machine learning or artificial intelligence.

Regarding the feedback the user may provide, there may be a number of options for the user to choose from, through the Ul.

During calibration, for a specific sensation, the selection of the electrodes to be activated, and the stimulation signal parameters may be independent: for example, first the electrodes may be selected, and then the stimulation signal parameters, applied to these electrodes may be determined.

In this way, a more accurate combination of electrodes and signals may be determined for an intended user sensation. Furthermore, for each different individual, a specific intended user sensation may end up having different combinations of electrodes and signals, creating a tailor-made set-up for each different user.

In an example, the system may implement machine learning algorithm for calibrating the combination of electrodes and signals corresponding to an intended user sensation. The machine learning algorithm may be a supervised training algorithm wherein different electrode configurations or patterns and different electrical stimulations parameters (e.g., amplitude, frequency, and pulse-width) may be used as training data sets, in order to evoke a natural somatotopic sensation, i.e., intended user sensation. The outputs of the algorithm may be then set based on the data related to the response of the user to each combination used at the input training data set.

Examples of supervised machine learning driven calibration cycle of the system herein disclosed comprise reinforcement learning in which the machine learning algorithm is trained using a training data set obtained from a large number of subjects whose perceived sensations in response to TENS nerve stimulation were collected through the User Interface. Additionally, the stimulation signal parameters and/or electrodes used to stimulate the subjects under different stimulation conditions, and perceived sensations were stored in a look-up table to be used as predetermined stimulation signal parameters to start the calibration process, according e.g. to demographic, physiological and other data of the subject.

The controller may be further configured to calibrate the system for a plurality of tactile and/or proprioceptive intended user sensations.

The controller may be further configured to employ the data stored in the storage device after the system is calibrated for a user, in order to generate intended somatotopic sensations in the user.

The controller may be configured to receive readings from at least one external sensor, and to control the stimulation module, depending on the received sensor readings, to stimulate at least one nerve and produce at least one intended somatotopic sensation in the user.

The controller may be configured to control the stimulation module in order to modulate, over time, the stimulation of one or more nerves and the production of somatotopic sensations, in response to the readings over time of a plurality of external sensors.

A kit comprising a system for somatotopic sensations using TENS electrodes in the lower limbs, and at least one external sensor in communication with the controller may also be provided. The sensor may be connected to the controller, and the controller may be configured to control the stimulation module to generate a predetermined somatotopic sensation by applying electrical signals to a group of electrodes based on the stored data within the storing device. That is, in day-to-day use, after calibration has been performed, the controller may send electrical signals to a group of electrodes which have been previously stored as corresponding to an intended user sensation. In this case, the generation of the predetermined somatotopic sensation may be triggered by readings of the at least one external sensor. For example, a sensor sensing a specific part of the sole of a foot of the user may be interpreted by the controller as the tip of the foot touching the floor and the ankle flexing upwards. Such type of movement may be related to a specific predetermined intended user sensation (what the user should feel when making that movement with the foot), which in turn may be stored in the storing device, and may have a set of electrodes and types of signals to be sent to the electrodes, which in turn may induce the intended sensation of the foot bending in such a way. Therefore, the system is able to, by recognizing, for example, a type of movement (or resting position, such as standing), send an electrical signal to the user, thus creating a somatotopic sensation which has been pre-calibrated, and thus the user may feel such sensation, either in the case where the limb that should be performing the movement is not able to send a signal to the user (suffers a neuropathy) or in the case where there exist limb, and the sensor may be placed, for example, in an artificial limb such as, for example, a foot prosthesis.

In this way, a natural sensation restoration is achieved by using the abovementioned features. The possibility to automatically select both the precise location of stimulation and the appropriate signal parameters allows the system to create complex stimulating policies to target specific sensations, and, even simultaneously, optimize their naturalness based on the information collected from external sensors (e.g., sensorized insoles). In some examples, an AI or machine learning routine implemented by the system of the present disclosure may allow for intelligent stimuli modulation during gait to account for changes in electrode impedance and position at different gait phases, affecting perceived sensation.

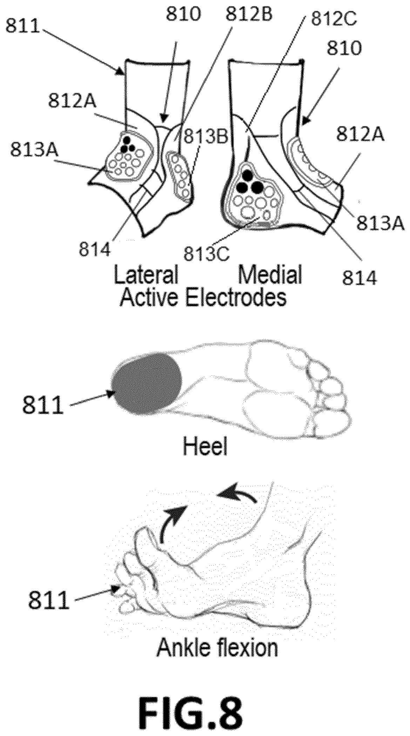

Electrodes from different arrays may be simultaneously stimulated to evoke several somatotopic sensations at the same time, e.g. ankle flexion and heel touch as it is explained further below. The configuration may be pre-programmed, provided in a look-up table, or similar. For example, if the external sensors detect only heel pressure, this may be recognized or programmed to mean that the ankle is flexed and the user is in a specific phase of the gait, and in response the controller may apply the calibrated stimulations for heel pressure and for ankle flexion, as explained with reference to figures 8, 9 and 10 below. This may be the result of a pre-programming, where each combination of external sensors readings may be associated with somatotopic sensations to be evoked.

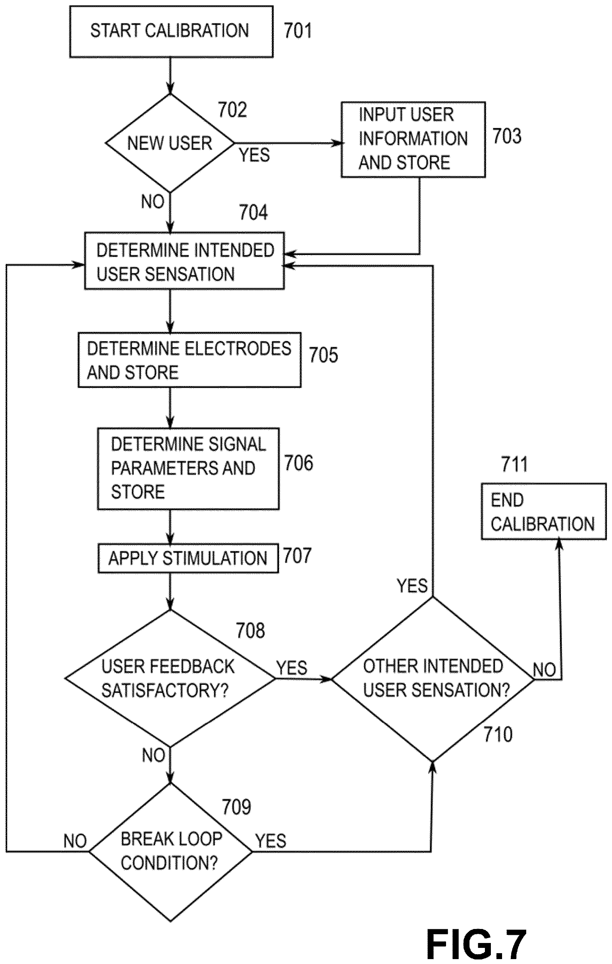

Figure 7 depicts a flowchart of an example of the calibration of an example of the system according to the present disclosure, as depicted in figure 6. More precisely, the calibration of the system starts in step 701, when the user indicates to start calibration through the UI 125. Then, in step 702 the system asks the user if he/she has previously calibrated the system. In case the user has not previously calibrated the system, in step 703 the user inputs data relating to personal information (which may be further used as a starting point for the system to perform the calibration) and the data is stored within the storage unit 123. In case the user has previously calibrated the system, the system may already have personal information of the user stored within the storage unit 123.

In step 704, an intended user sensation is determined, the sensation for which the calibration will be performed (for example, heel touching the floor or flexed ankle). Then, in step 705, the electrodes to be used in the nerve stimulation are determined by the controller 122 and stored within the storage unit 123, and in step 706, the stimulation signal parameters corresponding to the electrical signal to be applied to the determined electrodes are also determined by the controller 122, and stored within the storage unit 123.

In case this is the first cycle of the calibration for the intended user sensation, the controller 122 may select both the electrodes and the stimulation signal parameters to be used as a starting point for the calibration, based on the user information (e.g. demographic data, physiological data, or other). It may also take into account which type of intended user sensation it is being calibrated. Predetermined electrodes and stimulation signal parameters may also be stored within the storage unit 123, for each specific intended user sensation, to be retrieved by the controller and generically used as a start point for the calibration of the system for such sensation. If a look-up table is available as described above, based on the results of tests previously run with a number of subjects, the predetermined stimulation signal parameters may be those obtained from subjects with similar demographic features and for the same type of intended user sensation.

Furthermore, in case the system uses a Machine Learning algorithm, depending on the specific configuration of said algorithm, the controller 122 may choose a predetermined set of electrodes and stimulation signal parameters, or it may even start with a random set of electrodes and parameters, to be used as a starting point for the calibration of the system.

In case this is a subsequent cycle of the calibration of the determined user sensation, the controller may determine both the electrodes and the stimulation signal parameters based on data gathered from previous calibration cycles (for example, the electrodes and parameters used in the previous calibration cycle, whether using a Machine Learning Algorithm or not).

In step 707, the nerve stimulation is applied by sending to the determined electrodes an electrical signal according to the determined stimulation signal parameters, using the stimulator 62 to generate the electrical signal, and switch 63 to switch the signal and send it to the corresponding electrode array 61A, 61B or 61C.

In step 708, user feedback related to the sensation experienced by the user is received through UI 125, and based on such feedback, if it sufficiently satisfactory (i.e., the feedback is substantially similar to the intended user sensation), the calibration for the determined user sensation is considered to be finished. In such case, in step 710 the controller 122 may ask through UI 125 if a further intended user sensation may be calibrated. In case that a further intended user sensation may be calibrated, the controller 122 may go back to step 704 and determine a further user sensation to be calibrated.

In case that the user feedback is not satisfactory, step 709 may be performed wherein controller 122 may determine if a break loop condition is met. Such break loop condition may comprise, for example at least one of: a predetermined time, frequency, charge or current higher than a predetermined threshold. For example, if the frequency is higher than 200 Hz or the charge is higher than 20 µC the break loop condition is met.

If the break loop condition is not met, the controller may go back to step 704 in order to perform a further cycle of the calibration, either for the same intended user sensation (thus performing a subsequent cycle in the calibration of such sensation), or to change the intended user sensation (it may not be working properly for the user, and thus the user may want to try a different one, for example).

If the break loop condition is met, the controller 122 may ask the user if another intended user sensation may be calibrated in step 710. In case of positive answer, as previously described, the controller may go back to step 704. In case of negative result, the calibration may be ended in step 711.

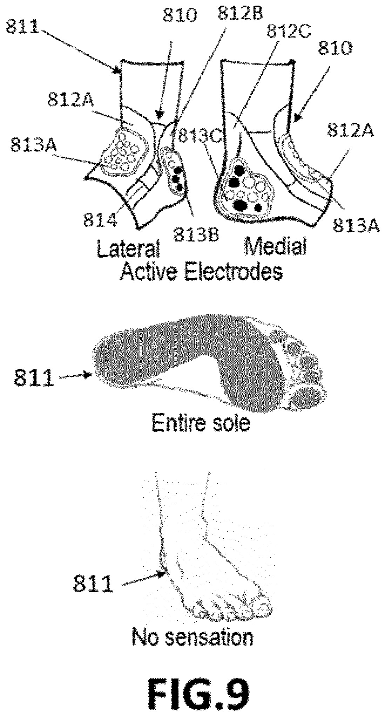

Figures 8 and 9 partially show an example system for producing somatotopic sensations using Transcutaneous Electrical Nerve Stimulation (TENS) in a foot 811. Foot 811 is shown from different perspectives, a lateral perspective, a medial perspective and a bottom perspective showing the sole of the foot 811. Figure 8 shows a wearable device 810 of the system, attached to the foot 811, the wearable device 810 comprising three units 812A, 812B and 812C, e.g., as in Figures 1 and 2 above, each unit comprising a corresponding array 813A, 813B, and 813C of TENS electrodes. In the particular case of figure 8, the arrays are shown as pads containing the electrodes, the electrodes represented by circles, but other solutions may be adopted. The electrodes are to be associated with at least one nerve of the foot 811, e.g., attached to the corresponding unit such as to be arranged as in Figures 5A, 5B and 5C once the wearable device 810 is attached to the foot 811. The wearable device 810 further comprises adjustable fasteners 814, e.g. Velcro straps, to attach the units together and to the foot 811. A stimulation module, not shown in figure 8, is in communication with the electrodes as disclosed in the examples above, and is configured to generate and transmit electrical signals to be applied to the electrodes. A controller (not shown in Figure 8), in communication with each electrode in the arrays 813A, 813B, and 813C of electrodes, is configured to select at least one pair of electrodes of at least one array of electrodes, to select stimulation signal parameters to be applied to the selected at least one pair of electrodes, and to control the stimulation module to generate and send an electrical signal with the selected stimulation signal parameters to the selected at least one pair of electrode. That is, the controller is configured to control the stimulation module to stimulate at least one nerve associated with an array of electrodes. The stimulation signal parameters may comprise stimulus intensity, frequency and pulse-width.

The system as disclosed herein and comprising wearable device 810 of figure 8, may operate in a calibration mode. In calibration mode, the controller learns which combination of electrodes and stimulation signal parameters may evoke or elicit an intended user sensation to a user. In other words, the controller calibrates the system by finding a combination of electrodes and stimulation signal parameters which may produce an intended user sensation to a user, e.g. within a certain range of tolerance. The intended sensation to be calibrated may be e.g. selected from a list of intended user sensations. The intended user sensation may be a somatotopic user sensation, such as tactile and/or proprioceptive user sensation. The calibration may be performed for a plurality of intended user sensations, e.g. one after the other.

For example, and with reference to figures 8 and 9, the system may be calibrated, for a specific user, to be able to apply stimulation signals to induce, for example, a somatotopic heel pressure sensation (figure 8, middle drawing) and/or a somatotopic ankle flexion sensation (figure 8, bottom drawing), and/or a somatotopic entire sole pressure sensation (figure 9). For this purpose, once the user has the wearable device attached to the foot as shown in Figures 8 and 9, in calibration model the controller may operate the iterative process described in detail in figure 7, in order to determine, for each of said intended sensations, at least one pair of electrodes of at least one array of electrodes 813A, 813B, and or 813C and select signal parameters such as intensity, frequency and pulse-width, which are suitable to induce the intended sensations in the user.

As explained for figure 7, during calibration, if the sensation experienced by the user when a certain stimulus is applied to certain electrodes coincides with or is similar to (within a range of tolerance) the intended user sensation, e.g. ankle flexion, heel pressure, or entire sole pressure, the system may store the set of calibration variables, e.g., intended user sensation, data related to the selected electrodes, and stimulation signal parameters.

Otherwise, if the sensation experienced by the user is not ankle flexion and/or is not heel pressure and/or is not entire sole pressure, then the calibration variables are not stored, and the system may iterate or repeat the calibration by changing the calibration variables until the response of the user to the nerve stimulation is (or is comprised within a range of tolerance from) the intended user sensation. or until other loop-breaking conditions are met, for example, a time limit.

In the example of figures 8 and 9, as a result of the calibration the controller may determine, and store, that for a specific individual the three electrodes colored in black of array 813C (figure 8) must be stimulated with first determined stimulation signal parameters, for inducing a somatotopic heel pressure sensation; the three electrodes colored in black of array 813A (figure 8) must be stimulated with second determined stimulation signal parameters for inducing a somatotopic ankle flexion sensation; and the three electrodes colored in black in array 813B (figure 9), plus at the same time the four electrodes colored in black in array 813C (figure 9), must be stimulated with third determined stimulation signal parameters, for inducing a somatotopic entire sole pressure sensation. For example, in arrays 813A and 813B, the upper electrode colored in black may be chosen as cathode, and the remaining two electrodes of each array may be chosen as anode; in array 813C the two upper electrodes colored in black may be chosen as cathode, and the lower two electrodes may be chosen as anode.

The calibration of the system for the user may be repeated for other tactile and/or proprioceptive intended user sensations.

In operation, e.g. during the user's normal life and/or activity, the system comprising the wearable device 810 of figures 8 and 9 may operate in a stimulation mode.

In stimulation mode, what is applied to the user may be calibrated stimuli, with each stimulus intended to evoke a specific somatotopic sensation. The controller uses the stored calibration data (specific electrodes or arrays 813A, 813B and/or 813C, and specific signal parameters, for each intended sensation) to control the stimulation module to stimulate the nerves of the user and induce the somatotopic intended user sensations that may be required or convenient in each moment.

In some cases, TENS stimulations may be delivered, e.g. to treat pain, during moments of inactivity (e.g., while sleeping). The stimulation may be triggered by the user from the user interface, and/or may be pre-programmed at certain times. Some stimulation parameters, such as the pulse width of the electrical signal, may be pre-programmed, in case the user triggers the stimuli (pain relief, etc.) or may depend on readings from external sensors, when external sensors are used.

In other cases, the somatotopic intended user sensations to be induced may be determined and triggered by the controller based on readings of external sensors: for example, in the case of figures 8 and 9, external sensors in a shoe for detecting that the user is in a specific phase of a walking cycle. Stimulus generation may therefore be modulated, over time, through external sensors (e.g., foot insoles with pressure sensors, wearable extensometers, prosthesis joint encoders, or others) connected to the controller, such that the controller may detect moments of activity, and an Al-driven restoration of a natural somatosensory sensation may be performed during use of the system, and without expert intervention.

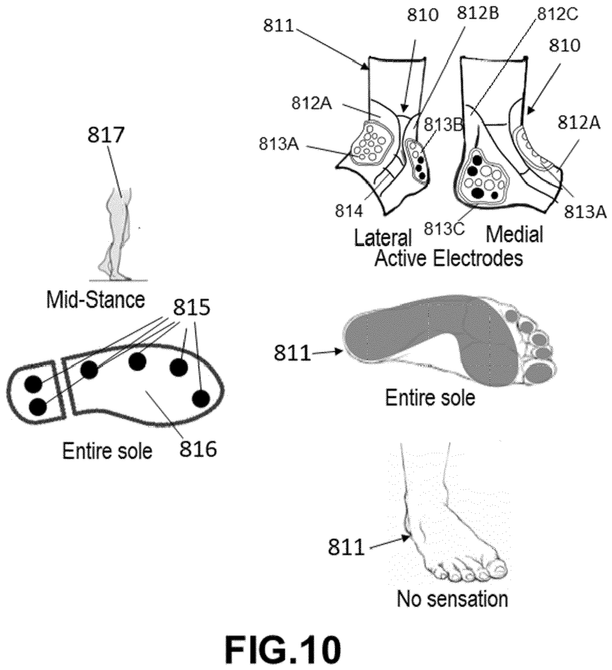

The system may operate in cooperation with one or more sensors which may sense an external pressure, temperature, or other inputs. The sensors may be in communication with the controller. In figure 10, for example, the controller (not shown) of a system such as that of figures 8 and 9 may be connected to force sensors 815 arranged in a sole 816 of a shoe or of a prosthesis. Other sensors, also referred to as "external sensors", may comprise foot insoles with pressure sensors, and/or wearable extensometers, and/or prosthesis and/or joint encoders, e.g. to detect joint rotations, e.g. an ankle flexion. As shown in figure 10, a user 817 may be walking wearing a shoe with the sole 816. In the shown mid-stance position, the sensors 815 of the sole 816 detect or sense that the entire sole is receiving an active pressure and the controller may control the stimulation module to generate a somatotopic sensation of pressure on the entire sole, by applying predetermined signals to pre-selected electrodes, colored in black of the arrays 813B 813C shown in figure 10, based on the calibration variables corresponding to the intended sensation to be induced. The application of the stimulation is therefore triggered by the controller based on the readings of the sensors 815.

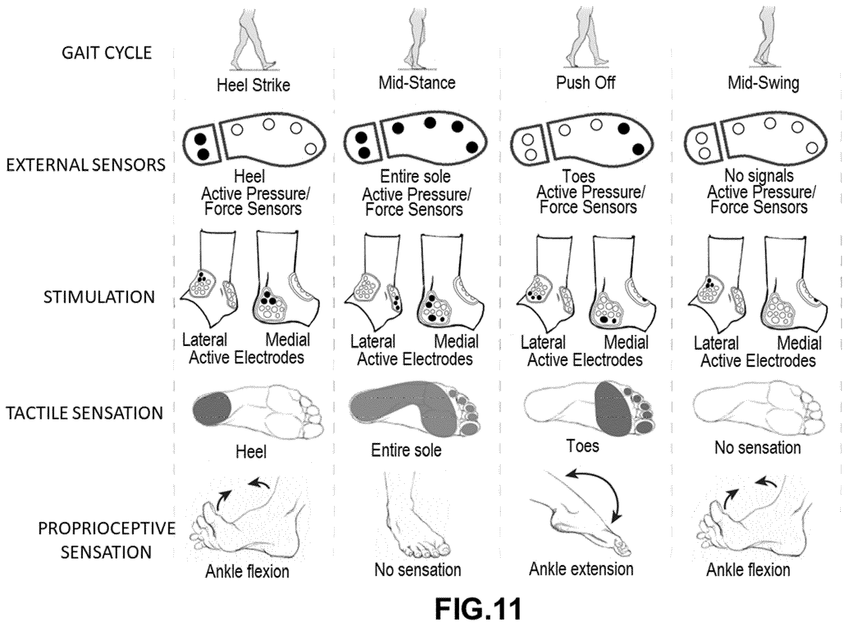

Figure 11 shows operation of the system as disclosed in relation to figures 8 to 10, during a complete gait cycle with four stages, using external sensors, such as sensors 815 shown in figure 10 in a prosthesis or sole (seen from below).

The system may have stored calibration variables for each of the represented intended user sensations: tactile sensations such as heel pressure, entire sole pressure, and pressure on the toes, and proprioceptive sensations such as ankle flexion and ankle extension. The sensors of the sole or external sensors in figure 11 are shown as colored in black when they are activated in response to a specific position or phase of the gait cycle.

Each phase of the gait cycle is represented in one of the columns of Figure 11. The first row of figure 11 shows the stage of the gait cycle. The following rows respectively show, for each cycle, and for the right foot: the external sensors that are activated; the electrodes to which stimulation is applied; the somatotopic tactile sensation induced by the stimulation; and the proprioceptive sensation induced by the stimulation.

The position of the sole and the ankle during each phase of the gait cycle is known: the association between the activation readings received by the controller from the external sensors (representative of the gait phase) and the somatotopic sensations to be induced (to mimic the natural sensations during the gait phase) may therefore be pre-programmed, for example in a look-up table or the like.

In the first column of figure 11, corresponding to the heel strike stage of the gait, the two external sensors that are positioned at the heel of the sole are activated by the pressure of the heel on the ground, and corresponding activation readings are received by the controller. Since in this first stage of the cycle the user heel contacts the ground and the user ankle is flexed, when the controller receives readings corresponding to the activation of these two sensors only, the controller may control the stimulation module to generate the somatotopic sensations "ankle flexion" and "heel pressure", by applying predetermined signals to predetermined electrodes (colored in black), predetermined according to the previous calibration of the system..

In the second phase of the gait cycle, when the user's right foot is in mid-stance, the sensors of the entire sole are activated. In this mid-stance phase, the whole user sole contacts the ground, and the user ankle is not flexed or extended, so no proprioceptive sensation needs to be induced for this stage. The controller may then control the stimulation module to generate the somatotopic sensation "pressure on the entire sole" by applying predetermined signals to the electrodes (colored in black) as shown in the second column of figure 11.

Still with reference to figure 11, in the third phase of the gait cycle, corresponding to the push off of the right foot, only the two sensors under the toes are activated. In this phase, only the front part of the foot or shoe contacts the ground, and the user ankle is extended. When the controller receives readings corresponding to the activation of these two sensors only, the controller may control the stimulation module to generate the somatotopic sensations "ankle extension" and "toes pressure".

Finally, in the fourth phase of the gait cycle, in the fourth column of figure 11 and corresponding to the mid-swing of the gait, in which the user's right foot is raised and the ankle is again flexed, no sensors are activated because there is no contact between the shoe and the ground. Therefore, when at this point of the cycle the controller receives no activation readings from the sensors, the controller may control the stimulation module to generate only the somatotopic sensations "ankle flexion".

In the context of a system as described with reference to figures 8-11, a stimulus generation may be modulated overtime through external sensors, and commands may be transferred to a controller for generation of the stimulus. During a walking cycle the external sensors may record changes of force/pressure under a foot. This information may be used to biomimetically apply neural stimulations. Thus, activation and deactivation of selected electrodes in the arrays, with suitable stimulus intensity, frequency and pulse-width may be used to restore natural tactile and proprioceptive sensations in the user.

The system as explained in the present disclosure may allow for Al-driven calibration of electrode selection in the arrays and stimulation signal parameters, for each somatotopic sensation to be induced, and for intelligent modulation of the stimulation over time using external sensory device inputs.

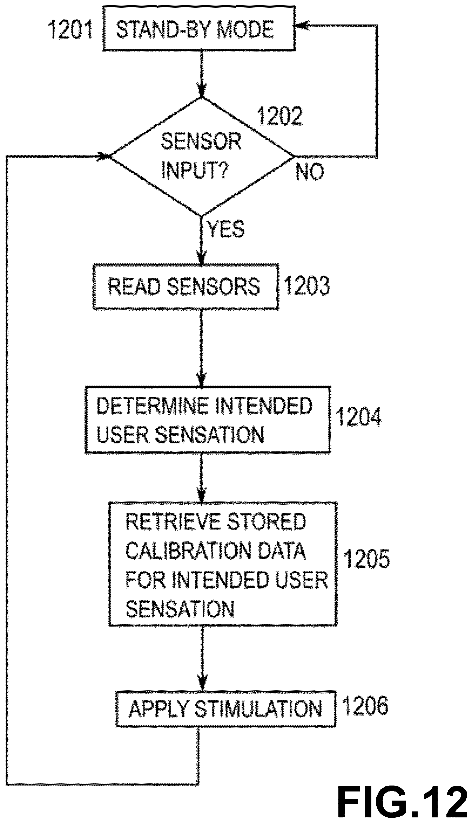

Figure 12 depicts a flow chart of an example of operation of the system according to figure 6 during user activity, after a calibration of the system has been performed. This may be for example the operation if the system during a gait cycle, as described in relation to figure 11.

More precisely, in step 1201 the system is in standby mode. The stimulation mode is triggered when, in step 1202, a sensor input is sent by the sensor 60 to the controller 122, triggering the performance of step 1203, wherein the sensor is read. In step 1204, the controller recognizes, based on the readings of the sensor 60, which of the pre-calibrated intended user sensations are to be induced in the user. In step 1205, the stored calibration data corresponding to the intended user sensation to be induced is retrieved from the storage unit 123. More precisely, the selected electrodes and the stimulation signal parameters corresponding to said intended user sensation are retrieved, which are used to apply a stimulation by generating and sending electrical signals to the selected electrodes, in step 1206. Therefore, in response to the trigger from the sensor, controller 122 stimulates the corresponding electrodes, and goes back to step 1202, in case the sensor keeps sensing a movement or situation where the user should be stimulated again, checking if it is the same intended user sensation or a different one, in order to apply the corresponding stimulation to the user.

Methods corresponding to the operation of a system as disclosed herein are also provided in the present disclosure, for example methods corresponding to flowcharts depicted in figures 7 or 12.

More generally, the present disclosure provides a method for producing somatotopic sensations using Transcutaneous Electrical Nerve Stimulation (TENS) in the lower limbs of a user, comprising:
- providing, around a joint of a lower limb of a user, a wearable device comprising at least two units, each unit comprising an array of TENS electrodes to be associated with at least one nerve of the lower limb;
- adapting the wearable to the user by attaching the at least two units to each other with adjustable fasteners;
- stimulating at least one nerve associated with an array of electrodes, by generating and applying an electrical signal to at least one pair of electrodes of the array of electrodes.

In some embodiments, the method may further comprise a calibration process to calibrate the system for a specific user and for at least one somatotopic intended sensation to be induced in the user. The calibration process may comprise, for each somatotopic intended sensation, an iterative process comprising a plurality of calibration cycles wherein stimulation signals with selected signal parameters are applied to selected electrodes, the selection of electrodes and signal parameters being successively modified in each calibration cycle based on user feedback to the stimulation signals. For example, the calibration process may be as in figure 7.

The method may then comprise, during use of the wearable device by the user, inducing somatotopic sensations in the user using, for each somatotopic sensation, the selected signal parameters and selected electrodes obtained in the calibration process.

A stimulation for inducing a somatotopic sensation in the user, by generating and applying an electrical signal to at least one pair of electrodes, may be triggered by a controller based on the readings of at least one external sensor, or may be triggered by a user order provided through a user interface.

Embodiments of the method may be performed with any system claimed or disclosed herein for producing somatotopic sensations using Transcutaneous Electrical Nerve Stimulation in the lower limbs.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A system for producing somatotopic sensations using Transcutaneous Electrical Nerve Stimulation (TENS) in the lower limbs, the system comprising:
- a wearable device to be attached to a lower limb of an individual, the wearable device comprising:
• at least two units, each unit comprising an array of TENS electrodes to be associated with at least one nerve of the lower limb; and
• adjustable fasteners to attach the units together and to the lower limb;
- a controller in communication with a stimulation module which is connected with the electrodes for generating electrical signals to be applied to the electrodes, the controller being configured to:
• select at least one pair of electrodes of at least one array of electrodes, and to select stimulation signal parameters to be applied to the selected at least one pair of electrodes; and
• control the stimulation module to generate and send, to the selected at least one pair of electrodes, an electrical signal based on the selected stimulation signal parameters, to stimulate at least one nerve associated with the array of electrodes comprising the selected at least one pair of electrodes.

2. System according to claim 1, wherein at least one unit of the wearable device further comprises a joint anchor, shaped to position the unit with respect to a joint of the lower limb.

3. System according to claim 2, wherein the joint anchor comprises a pad arranged along a contour portion of the unit, the pad having a shape matching at least part of a joint of the lower limb.

4. System according to any of claims 2 or 3, wherein each unit of the wearable device comprises one or more joint anchors, wherein joint anchors of different units are configured to at least partially surround between them a joint of the lower limb, when the wearable device is attached to the lower limb.

5. System according to any of claims 1 to 4, further comprising a switch, the switch belonging to the stimulation module and being connected to the controller, and a plurality of transmission lines individually connecting each electrode to the switch, wherein the switch is configured to send to at least one pair of electrodes, according to a selection received from the controller, an electrical signal generated by a stimulator of the stimulation module.

6. System according to claim 5, wherein the switch is attached to one of the units of the wearable device, and each unit of the wearable device comprises transmission lines connected to the electrodes of the arrays of electrodes of the unit.

7. System according to any of claims 1 to 6, wherein the wearable device is intended to be worn around an ankle of a user and comprises three units, each comprising one array of electrodes, wherein the array of electrodes of a first unit is to be associated with the Peroneal Nerve and comprises between 2 and 9 electrodes, the diameter of each electrode being between 1 and 4 cm, the array of electrodes of a second unit is to be associated with the Tibial Medial Nerve and comprises between 2 and 17 electrodes, the diameter of each electrode being between 1 and 4 cm, and the array of electrodes of a third unit is to be associated with the Calcaneal Nerve and comprises between 2 and 9 electrodes, the diameter of each electrode being between 1 and 3 cm.

8. System according to any of claims 1 to 6, wherein the wearable device is intended to be worn around the knee of a user and comprises three units, each comprising one array of electrodes, wherein the array of electrodes of a first unit is to be associated with the Frontal Tibial Nerve and comprises between 2 and 6 electrodes, the diameter of each electrode being between 1 and 3 cm, the array of electrodes of a second unit is to be associated with the Common Peroneal Nerve and comprises between 2 and 6 electrodes, the diameter of each electrode being between 1 and 3 cm, and the array of electrodes of a third unit is to be associated with the Posterior Tibial Nerve and comprises between 2 and 6 electrodes, the diameter of each electrode being between 1 and 2 cm.

9. System according to any of claims 1 to 8, further comprising a User Interface in communication with the controller, and a storage device connected to the controller, wherein the controller is further configured to calibrate the system for one user, for producing at least one intended user sensation, by performing, for a specific intended user sensation, a first calibration cycle, the first calibration cycle comprising:
- selecting at least one pair of electrodes of at least one array of electrodes;
- causing a nerve stimulation by applying an electrical signal to the selected at least one pair of electrodes to stimulate at least one nerve, the electrical signal being based on predetermined stimulation signal parameters;
- receiving from the User Interface feedback data relating to a sensation experienced by the individual in response to the nerve stimulation; and
- based on the received feedback data, storing in the storage device:
• the intended user sensation,
• data related to the selected at least one pair of electrodes; and
• the predetermined stimulation signal parameters.

10. System according to claim 9, wherein the controller is further configured to perform subsequent calibration cycles, wherein each subsequent calibration cycle comprises:
- causing a nerve stimulation by applying an electrical signal of selected stimulation signal parameters to at least one selected pair of electrodes of the array of electrodes, wherein the at least one selected pair of electrodes and/or the stimulation signal parameters are different from those of a previous cycle,
- receiving from the User Interface feedback data relating to a sensation experienced by the individual in response to the nerve stimulation; and
- based on the received feedback data, storing in the storage device:
• the intended user sensation,
• data related to the at least one selected pair of electrodes; and
• the stimulation signal parameters.

11. System according to claim 10, wherein the controller is further configured to calibrate the system for a plurality of tactile and/or proprioceptive intended user sensations.

12. System according to any of claims 9 to 11, wherein the controller is further configured to employ the data stored in the storage device after the system is calibrated for the user, in order to generate intended somatotopic sensations in the user.

13. System according to claim 12, wherein the controller is also configured to receive readings from at least one external sensor, and to control the stimulation module, depending on the received sensor readings, to stimulate at least one nerve and produce at least one intended somatotopic sensation in the user.

14. System according to claim 13, wherein the controller is configured to control the stimulation module in order to modulate, over time, the stimulation of one or more nerves and the production of somatotopic sensations, in response to the readings over time of at least one external sensor.

15. A kit comprising a system according to any of claims 13 to 14, and at least one external sensor in communication with the controller.
